Europäisches Patentamt

(19) European Patent Office

Office européen des brevets

(11) Publication number: **0 103 637**
**B1**

## (12) EUROPEAN PATENT SPECIFICATION

(45) Date of publication of patent specification: **29.07.87**

(21) Application number: **83901462.8**

(22) Date of filing: **15.03.83**

�ououtINTERNATIONAL application number:
**PCT/US83/00347**

(87) International publication number:
**WO 83/03190 29.09.83 Gazette 83/23**

(51) Int. Cl.⁴: **A 61 B 5/00**

(54) **SUBARACHNOID BOLTS.**

(30) Priority: **15.03.82 US 358444**

(43) Date of publication of application:
**28.03.84 Bulletin 84/13**

(45) Publication of the grant of the patent:
**29.07.87 Bulletin 87/31**

(84) Designated Contracting States:
**AT BE CH DE FR GB LI LU NL SE**

(56) References cited:
**EP-A-0 074 037**
**FR-A-2 384 482**
**NL-A-7 801 416**
**US-A-4 062 354**
**US-A-4 186 728**

(73) Proprietor: **SWEENEY, Paul L. Jr.**
**20 Roosevelt Drive**
**Laurel Springs NJ 08021 (US)**

(72) Inventor: **LETTERIO, Fred**
**3334 Fordham Road**
**Philadelphia, PA 19114 (US)**

(74) Representative: **Weydert, Robert et al**
**OFFICE DENNEMEYER S.à.r.l. P.O. Box 1502**
**L-1015 Luxembourg (LU)**

Courier Press, Leamington Spa, England.

## Description

The present invention concerns a subarachnoid bolt for use in measuring intracranial pressure. Several devices have been utilized to perform such measurements. These include intraventricular catheters, subarachnoid bolts and solid state or implantable transducers. This invention relates to improvements in subarachnoid bolts.

Subarachnoid bolts for measuring or monitoring intracranial pressure have been known for some time. One of the more widely utilized bolts, commonly referred to as a "Philly" bolt, is comprised essentially of stainless steel or similar material and includes an external screw thread at its lower end which is intended to be screwed into a twist drill hole formed in a patient's skull. The extreme end of the bolt enters the subarachnoid space over the cerebral hemisphere.

EP—A—0074 037 discloses a subarachnoid bolt for use in measuring intracranial pressure including a lower bolt half having an enlarged flange and a coaxially arranged tubular member extending downwardly from said flange, at least a part of said tubular member being radially expandable so that the same can be secured to the patient's skull and an upper bolt half adapted to be screwed to the top of said lower bolt half. (That document forms a part of the prior art under Article 54(3) and (4) EPC only, with respect to all the designated Contracting States, except Luxembourg.)

While known subarachnoid bolts have met with some success, they have also suffered from many problems particularly with patients with very thin skulls such as neonatal patients. With such patients and in many other cases, it is extremely difficult and sometimes impossible to secure the bolt to the skull by screwing the same thereto.

The present invention overcomes the defects of the prior art known to Applicant and provides a subarachnoid bolt which can be easily secured to substantially any skull.

The bolt of the present invention includes a lower bolt half having an enlarged flange and a coaxially arranged tubular member extending downwardly from said flange, said tubular member being adapted to be inserted into a substantially complementary shaped hole in a patient's skull with said flange overlying the outer surface of the skull around said hole, at least a part of said tubular member being radially expandable so that the same can be secured to the patient's skull;

means on said lower bolt half allowing the same to be held to prevent rotation thereof;

an upper bolt half adapted to be screwed to the top of said lower bolt half, and means for causing said at least part of said tubular member to radially expand when said two bolt halves are screwed together.

In one embodiment, the bolt is securely held in place when the free end of a tubular element carried by the upper bolt half enters the tubular member and cams the lowermost end thereof radially outwardly when the two bolt halves are screwed together. In a second embodiment, a sleeve on the lower bolt half bulges outwardly against the walls of the hole when the two bolt halves are screwed together.

For the purpose of illustrating the invention, there are shown in the accompanying drawings forms which are presently preferred; it being understood that the invention is not intended to be limited to the precise arrangements and instrumentalities shown.

Figure 1 is a perspective view of a subarachnoid bolt constructed in accordance with the principles of the present invention;

Figure 2 is an exploded perspective view of the bolt shown in Figure 1;

Figure 3 is a cross-sectional view of the lower bolt half inserted in a hole in a patient's skull;

Figure 4 is a cross-sectional view of the bolt secured to a patient's skull;

Figure 5 is a perspective view of a second embodiment of a subarachnoid bolt according to the invention;

Figure 6 is an exploded perspective view of the bolt shown in Figure 5;

Figure 7 is a cross-sectional view of the lower half of the bolt of Figure 5 inserted in a hole in a patient's skull; and

Figure 8 is a cross-sectional view of the bolt of Figure 5 secured to a patient's skull.

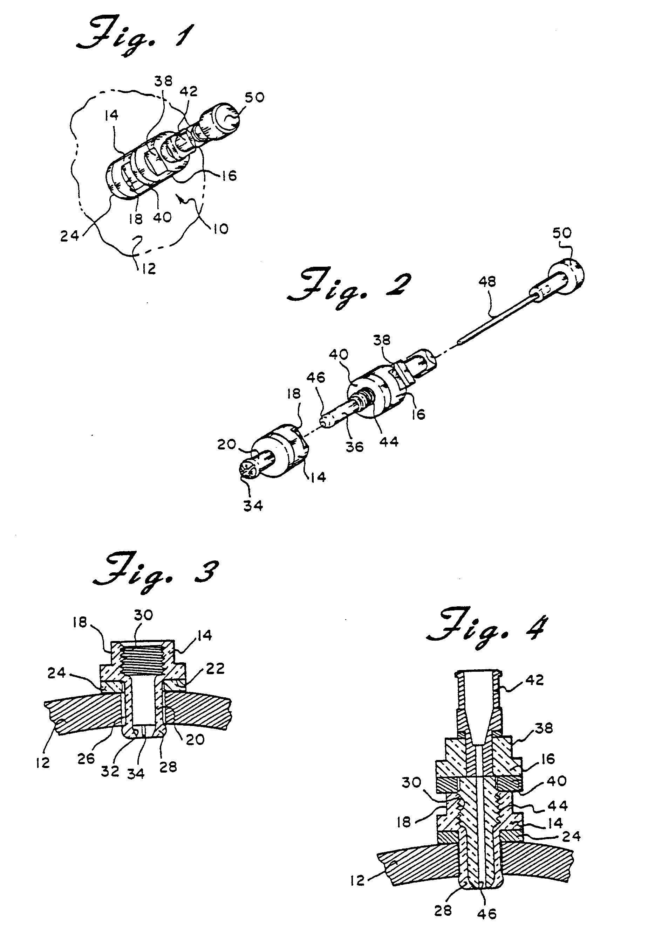

Referring now to the drawings in detail wherein like reference numerals have been used throughout the various figures to designate like elements, there is shown in Figure 1 a first embodiment of a subarachnoid bolt constructed in accordance with the principles of the present invention and designated generally as 10. In Figure 1, bolt 10 is shown secured to the skull 12 of a patient.

Bolt 10 is comprised of two parts: a lower bolt half 14 and an upper bolt half 16. As shown most clearly in Figures 2 and 3, lower bolt half 14 is essentially circular in cross section except for the upper portion thereof which is squared as shown at 18 to form a means by which a wrench or similar tool can hold the lower bolt half 14 to prevent the same from rotating. The reason for this will become more readily apparent hereinafter.

The lower section of lower bolt half 14 is reduced in diameter and forms a tubular member 20 which is coaxially aligned with the remaining parts of the bolt. An enlarged flange 22 is thereby formed above the tubular member 20. A sealing washer 24 rests against the flange 22 and around the upper part of the tubular member 20.

As shown most clearly in Figure 3, the tubular member 20 is adapted to be inserted into a substantially complementary shaped hole 26 in a patient's skull 12. The length of the tubular member 20 is selected so that the lowermost end 28 thereof which is slightly bulbous lies just below the lowermost surface of the skull 12. The flange 22 and washer 24 overlie the outer surface of the skull 12 around the hole 26.

Lower bolt half 14 is substantially hollow, having a channel passing entirely therethrough. An internal screw head 30 is formed in the uppermost part and the inner surface of the lowermost end 28 is substantially conically shaped as shown at 32. The wall of the lowermost end 28 of the tubular member 20 also includes a plurality of axially extending slits 34 therein. In the preferred embodiment of the invention, four such slits are shown (see Figure 2). This is by way of example only as the invention could function with fewer or more such slits. The purpose of the slits 34 is to weaken the wall of the lowermost end portion 28 slightly so that the same can be flexed outwardly when the bolt is in use as will be explained more clearly hereinafter. With the end portion 28 flexed outwardly, the bulbous portion forces against the undersurface of the skull 12 to retain the bolt in place.

The details of the upper bolt half 16 are shown most clearly in Figures 2 and 4. As with the lower bolt half 14, the upper bolt half 16 has a main body portion with a concentrically arranged tubular element 36 extending downwardly therefrom. A squared section 38 similar to section 18 also allows the upper bolt half to be held by a wrench or similar tool so that the same can be turned relative to the lower bolt half. A washer 40 similar to washer 24 extends around the uppermost part of the tubular element 36. The uppermost part of the upper bolt half 16 carries a coaxially arranged adapter or connector 42 which is utilized for connecting the bolt to remotely located sensing and/or measuring equipment.

An external screw thread 44 is formed on the upper end of the tubular element 36. This screw thread 44 is complementary to the screw thread 30 on the lower bolt half 14 so that the two bolt halves can be secured together with the tubular element 36 entering the interior of the lower bolt half 14. The forwardmost end 46 of the tubular element 36 is also substantially conically shaped and as the two bolt halves are screwed together, this end 46 moves downwardly and pushes against the conical inner surface 32 to cam the lower end 28 of the lower bolt half 14 outwardly to secure the bolt in place. A conventional pin 48 with handle 50 may also be utilized to seal the interior of the bolt 10 whenever the same is not being utilized for measuring intracranial pressure.

Bolt 10 is used in the following manner. A hole 26 is first drilled in the patient's skull 12 at the appropriate position. Bolt halves 14 and 16 are separated and tubular member 20 of bolt half 14 is passed through the hole 26 with the bulbous end 28 lying just below the surface of the skull 12 and the washer 24 and flange 22 lying above the skull for sealing the same. The tubular element 36 of the upper bolt half 16 is then inserted into the interior of the lower bolt half 14. A wrench is then used to hold the lower bolt half 14 while a second wrench or similar tool is used to turn the upper bolt half 16 so that the two bolt halves are threaded together. Eventually, the lowermost end 46 of the tubular element 36 will function as a cam to flex the lowermost end 28 of the tublar member 20 outwardly to secure the bolt in position on the skull as shown in Figure 4.

A second embodiment of the invention is illustrated in Figures 5—8. A subarachnoid bolt constructed in accordance with the principles of this embodiment of the invention is designated generally in Figure 5 as 110.

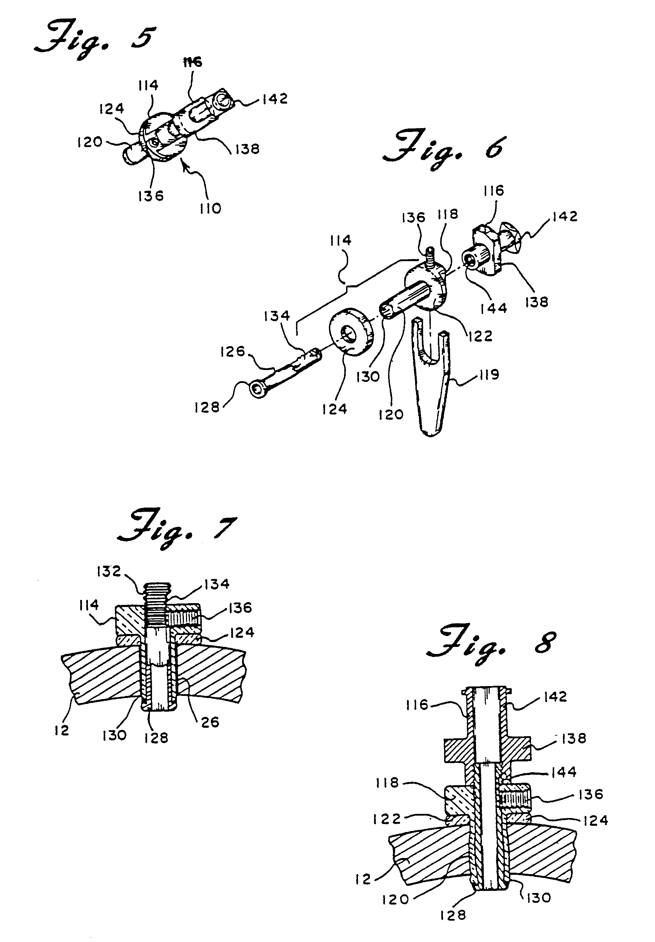

As with bolt 10, bolt 110 is comprised essentially of two parts: a lower bolt half 114 and an upper bolt half 116. As shown most clearly in Figures 6—8, lower bolt half 114 is essentially circular in cross section except for the upper portion thereof which is squared as shown at 118 to form a means by which a wrench or similar tool 119 can hold the lower bolt half 114 to prevent the same from rotating.

The lower section of lower bolt half 114 is reduced in diameter and forms a tubular member 120 which is coaxially aligned with the remaining parts of the bolt. An enlarged flange 122 is thereby formed above the tubular member 120. A sealing washer 124 rests against the flange 122 and around the upper part of the tublar member 120.

Extending upwardly through the tubular member 120 is a hollow screw 126. The lowermost portion of the hollow screw 126 has an enlarged head 128 which abuts the extreme lower edge 130 of the tubular member 120 which prevents upward movement of the hollow screw 126. Screw 126 is long enough to extend slightly above the upper surface of the lower bolt half 114 and has this upper portion threaded as shown at 132 in Figure 7. Adjacent the upper portion of the screw 126, one side thereof is flattened as shown at 134. This flattened portion 134 is engaged by a set screw 136 which passes through the side wall of the upper part of the lower bolt half 114. The set screw 136 does not tightly engage the flattened surface 134 of the screw 126. Rather, it is intended to prevent rotational movement of the screw 126 while allowing limited axial movement. The reasons for this will become more readily apparent hereinafter.

As shown most clearly in Figures 7 and 8, the tubular member 120 is adapted to be inserted into a substantially complementary shaped hole 26 in a patient's skull 12. The length of the tubular member 120 is selected so that the lowermost end including the lower portion 128 of the screw 126 is just below the lowermost surface of the skull 12. In this position, the flange 122 and washer 124 overlie the outer surface of the skull 12 around the hole 26 in substantially the same manner as the bolt 10 described in Figures 1—4.

The upper bolt half 116 is also substantially circular in cross section except for the squared section 138 which is similar in shape to the squared section 118 on the lower bolt half. The uppermost part of the upper bolt half 116 carries a coaxially arranged adapter or connector 142 which is utilized for connecting the bolt to remotely located sensing and/or measuring equipment. The lower end of the upper bolt half 116 has an internal thread 144 formed therein which is adapted to cooperate with the threaded portion 132 at the upper end of the screw 126.

Bolt 110 is used in the following manner. As with bolt 10, a hole 26 is first drilled in the patient's skull 12 at the appropriate position. Bolt halves 114 and 116 are separated and tubular member 120 of bolt half 114 is passed through the hole 26 with the lower end 128 of the screw 126 and the lower end 130 of the tubular member 120 lying just below the surface of the skull 12 and the washer 124 and flange 122 lying above the skull for sealing the same. The upper bolt half 116 is then screwed onto the lower bolt half by engaging the internal threads 144 with the external threads 132. Wrench 119 is utilized to hold the lower bolt half 114 in a stationary position while a second wrench or similar tool is used to turn the upper bolt half 116. Since the screw 126 is prevented from rotational movement by set screw 136, it is eventually drawn upwardly as the upper bolt half 116 is turned. Head 128 at the end of screw 126 engages the lower end 130 of the tubular member 120 and attempts to push this upwardly. Since the entire tubular member 120 cannot move upwardly, it eventually expands or bulges outwardly and seals tightly against the inner wall of the hole 26 in the skull 12, thus securing the bolt in position. This is clearly shown in Figure 8.

Any suitable materials may be utilized in the manufacture of the bolts 10 and 110. In the preferred embodiments, the adapters 42 and 142 and the screws 126 and 136 are comprised of stainless steel or the like while the remaining parts of the bolt are preferably made of relatively rigid plastic. The washers are, of course, preferably comprised of a somewhat softer material so that they will produce the desired sealing function. Furthermore, the tubular member 120 must be relatively rigid but flexible enough to flex outwardly to perform its sealing function.

The present invention may be embodied in other specific forms without departing from the scope of the invention, which is defined in the appended claims.

## Claims

1. A subarachnoid bolt (10,110) for use in measuring intracranial pressure including:

a lower bolt half (14,114) having an enlarged flange (22,122) and a coaxially arranged tubular member (20,120) extending downwardly from said flange (22,122), said tubular member (20,120) being adapted to be inserted into a substantially complementary shaped hole (26) in a patient's skull with said flange (22,122) overlying the outer surface of the skull around said hole (26), at least a part (28,128) of said tubular member (20,120) being radially expandable so that the same can be secured to the patient's skull;

means (18,118) on said lower bolt half (14,114) allowing the same to be held to prevent rotation thereof;

an upper bolt half (16,116) adapted to be screwed to the top of said lower bolt half, (14,114) and means (32,46/128,130) for causing said at least part (28,128) of said tubular member (20,120) to radially expand when said two bolt halves (14,16; 114,116) are screwed together.

2. The subarachnoid bolt according to claim 1 wherein said upper bolt half (16) has a downwardly extending tubular element (36) adapted to fit within said tubular member (20) of said lower bolt half (14) when said upper (16) and lower bolt halves (14) are screwed together.

3. The subarachnoid bolt according to claim 2 wherein the lowermost end (28) of said tubular member (20) is made to expand radially by the leading end (46) of said tubular element (36).

4. The subarachnoid bolt according to claim 3 wherein the lowermost end (28), of said tubular member (20) includes a plurality of axially extending slits (34) in the wall thereof.

5. The subarachnoid bolt according to claim 3 wherein the outer configuration of the lowermost end (28) of said tubular member (20) is slightly bulbous.

6. The subarachnoid bolt according to claim 3 wherein the inner surface (32) of the lowermost end (22) of said tubular member (20) is substantially conically shaped and functions as a cam surface.

7. The subarachnoid bolt according to claim 2 wherein said lower bolt half (14) has an opening in the upper surface thereof which is concentric with said tubular member (20) and which has an internal screw thread (30) therein and wherein the upper end of said tubular element (36) includes a complementary external screw thread (44) thereon.

8. The subarachnoid bolt according to claim 1, wherein said lower bolt half (114) includes a hollow screw (126) passing upwardly through the tubular member (120) and being exposed at the upper surface of the lower bolt half.

9. The subarachnoid bolt according to claim 8 wherein said upper bolt half (116) is adapted to be screwed to the exposed portion of said hollow screw (126).

## Patentansprüche

1. Subarachnoidalbolzen (10,110) zur Verwendung beim Messen des intrakraniellen Druckes, mit:

einen unteren Bolzenhälfte (14,114), die einen erweiterten Flansch (22,122) und ein koaxial angeordnetes rohrförmiges Teil (20,120), welches sich von dem Flansch (22,122) aus nach unten erstreckt, hat, wobei das rohrförmiges Teil (20,120) zum Einführen in ein im wesentlichen komplementär geformtes Loch (26) in der Schädeldecke eines Patienten vorgesehen ist, wobei der Flansch (22,122) über der äußeren Oberfläche der Schädeldecke um das Loch (26) zu liegen kommt und wobei wenigstens ein Teil (28,128) des rohrförmigen Teils (20,120) radial aufweitbar ist, so daß dasselbe an der Schädeldecke eines Patienten befestigt werden kann;

7 0 103 637 8

einer Einrichtung (18,118) an der unteren Bolzenhälfte (14,114), die gestattet, dieselbe festzuhalten, um deren Drehung zu verhindern;

einer oberen Bolzenhälfte (16,116), die zum Verschrauben mit dem oberen Ende der unteren Bolzenhälfte (14,114) vorgesehen ist, und einer Einrichtung (32,46; 128,130), die bewirkt, daß wenigstens ein Teil (28,128) des rohrförmigen Teils (20,120) sich radial aufweitet, wenn die Bolzenfälften (14,16; 114,116) zusammengeschraubt werden.

2. Subarachnoidalbolzen nach Anspruch 1, wobei die obere Bolzenhälfte (16) ein sich nach unten erstreckendes rohrförmiges Element (36) hat, das in das rohrförmige Teil (20) der unteren Bolzenhälfte (14) paßt, wenn die obere (16) und die untere Bolzenhälfte (14) zusammengeschraubt werden.

3. Subarachnoidalbolzen nach Anspruch 2, wobei das unterste Ende (28) des rohrförmigen Teils (20) durch das Vorderende (46) des rohrförmigen Elements (36) radial aufweitbar ist.

4. Subarachnoidalbolzen nach Anspruch 3, wobei das unterste Ende (28) des rohrförmigen Teils (20) mehrere sich axial erstreckende Schlitze (34) in dessen Wand aufweist.

5. Subarachnoidalbolzen nach Anspruch 3, wobei die äußere Konfiguration des untersten Endes (28) des rohrförmigen Teils (20) etwas kugelig ist.

6. Subarachnoidalbolzen nach Anspruch 3, wobei die innere Oberfläche (32) des untersten Endes (22) des rohrförmigen Teils (20) im wesentlichen konisch ist und als Nockenfläche dient.

7. Subarachnoidalbolzen nach Anspruch 2, wobei die untere Bolzenhälfte (14) eine Öffnung in ihrer oberen Oberfläche hat, die zu dem rohrförmigen Teil (20) konzentrisch ist und ein inneres Schraubengewinde (30) aufweist, und wobei das obere Ende des rohrförmigen Elements (36) ein komplementäres äußeres Schraubengeweinde (44) aufweist.

8. Subarachnoidalbolzen nach Anspruch 1, wobei die untere Bolzenälfte (114) eine Hohlschraube (126) aufweist, die aufwärts durch das rohrförmige Teil (120) hindurchführt und an der oberen Oberfläche der unteren Bolzenhälfte freiliegt.

9. Subarachnoidalbolzen nach Anspruch 8, wobei die obere Bolzenhälfte (116) zum Einschrauben in den freiliegenden Teil der Hohlschraube (126) vorgesehen ist.

**Revendications**

1. Boulon sous-arachnoïdien (10,110) destiné à être utilisé pour mesurer la pression intracrânienne, ce boulon comprenant:

une moitié inférieure (14,114) comportant une bride élargie (22,122) et un organe tubulaire coaxial (20,120) s'étendant vers le bas à partir de cette bride (22,122), cet organe tubulaire (20,120) étant conçu pour être introduit dans un trou de forme pratiquement complémentaire (26) pratiqué dans la boîte crânienne d'un patient, de telle sorte que la bride (22,122) recouvre la surface extérieure de la boîte crânienne autour de ce trou (26), au moins une partie (28,128) de l'organe tubulaire (20,120) étant radialement expansible, de telle sorte qu'il puisse être fixé à la boîte crânienne du patient;

un moyen (18,118) prévu sur la moitié inférieure (14,114) du boulon et permettant de maintenir ce dernier pour l'empêcher de tourner;

une moitié supérieure (16,116) conçue pour être vissée sur le sommet de la moitié inférieure (14,114) du boulon, ainsi qu'un moyen (32,46/ 128,130) destiné à provoquer une expansion radiale de la partie (28,128) de l'organe tubulaire (20,120) lorsque les deux moitiés de boulon (14,16; 114,116) sont vissées ensemble.

2. Boulon sous-arachnoïdien selon la revendication 1, dans lequel la moitié supérieure (16) de ce boulon comporte un élément tubulaire (36) s'étendant vers le bas et conçu pour venir s'adapter à l'intérieur de l'organe tubulaire (20) de la moitié inférieure (14) du boulon lorsque les moitiés de boulon supérieure (16) et inférieure (14) sont vissées ensemble.

3. Boulon sous-arachnoïdien selon la revendication 2, dans lequel l'extrémité inférieure (28) de l'organe tubulaire (20) est soumise à une expansion radiale par l'extrémité avant (46) de l'élément tubulaire (36).

4. Boulon sous-arachnoïdien selon la revendication 3, dans lequel l'extrémité inférieure (28) de l'organe tubulaire (20) comporte plusieurs fentes axiales (34) pratiquées dans sa paroi.

5. Boulon sous-arachnoïdien selon la revendication 3, dans lequel l'extrémité inférieure (28) de l'organe tubulaire (20) a une configuration extérieure légèrement bulbeuse.

6. Boulon sous-arachnoïdien selon la revendication 3, dans lequel la surface intérieure (32) de l'extrémité inférieure (22) de l'organe tubulaire (20) a une configuration pratiquement conique et fait office de surface de came.

7. Boulon sous-arachnoïdien selon la revendication 2, dans lequel la moitié de boulon inférieure (14) comporte, dans sa surface supérieure, une ouverture qui est concentrique par rapport à l'organe tubulaire (20) et qui est pourvue d'un filet de vis intérieur (30), un filet de vis extérieur complémentaire (44) étant formé sur l'extrémité supérieure de l'élément tubulaire (36).

8. Boulon sous-arachnoïdien selon la revendication 1, dans lequel la moitié de boulon inférieure (114) comprend une vis creuse (126) s'étendant vers le haut à travers l'organe tubulaire (120) et exposée à la surface supérieure de la moitié de boulon inférieure.

9. Boulon sous-arachnoïdien selon la revendication 8, dans lequel la moitié de boulon supérieure (116) est conçue pour être vissée à la partie exposée de la vis creuse (126).

Fig. 1

Fig. 2

Fig. 3

Fig. 4

Fig. 5

Fig. 6

Fig. 7

Fig. 8